(19) Europäisches Patentamt
European Patent Office
Office européen des brevets

(11) Publication number: 0 097 942
B1

(12) # EUROPEAN PATENT SPECIFICATION

(45) Date of publication of patent specification: **22.07.87**

(51) Int. Cl.[4]: **C 07 D 241/20,** A 61 K 31/495

(21) Application number: **83106229.4**

(22) Date of filing: **27.06.83**

(54) **Antiallergic pyrazine-bis(2-oxoglycine) compounds.**

(30) Priority: **30.06.82 US 393958**

(43) Date of publication of application:
**11.01.84 Bulletin 84/02**

(45) Publication of the grant of the patent:
**22.07.87 Bulletin 87/30**

(84) Designated Contracting States:
**AT BE CH DE FR GB IT LI LU NL SE**

(56) References cited:
**US-A-3 963 660**
**US-A-4 017 538**
**US-A-4 191 840**

(73) Proprietor: **THE DOW CHEMICAL COMPANY**
**2030 Dow Center Abbott Road P.O. Box 1967**
**Midland, MI 48640 (US)**

(72) Inventor: **Wood, Steven Glen**
**1786 North 80 East**
**Orem Utah 84057 (US)**

(74) Representative: **Sgarbi, Renato et al**
**Gruppo Lepetit S.p.A.**
**Patent & Trademark Department**
**34 Via Roberto Lepetit**
**I-21040 Gerenzano (Varese) (IT)**

EP 0 097 942 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

FIELD OF THE INVENTION

The invention is a group of compounds which inhibit release of mediators, such as histamine, which play a key role in allergic responses.

BACKGROUND OF THE INVENTION

The role of antigen/antibody reactions in effecting release of mediators from mast cells is discussed in U.S.P. 4,191,840 — which is directed to a group of antiallergic benzene oxamic acid compounds having mediator release inhibiting (MRI) activity. Pyridine dioxamic acid compounds useful in the treatment of allergy are disclosed in U.S.P. 3,963,660. Both types of the patented compounds are said to be orally administrable.

OBJECTS OF THE INVENTION

The primary object of the present invention is to provide new orally administrable antiallergy agents.

Another object is to provide MRI compounds of a type other than those disclosed in the foregoing '660 and '840 patents.

A further object is to provide an orally administrable MRI compound having a low $ED_{50}$ and exhibiting rapid onset of activity with a high level of inhibition.

A corollary object is to provide a novel process for the preparation of 2,6-pyrazinediyl dioxamic acid compounds substituted or unsubstituted in the 3- and 5-positions.

Additional objects will be made apparent by the following specifications and claims, to those knowledgeable in the art.

SUMMARY OF THE INVENTION

It has now been discovered that certain pyrazine oxamic acid compounds are orally administrable and have antiallergic activity. These compounds have the following formula:

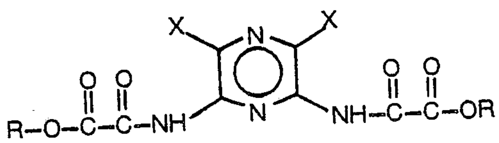

wherein

X is H, Br or Cl, the same in each occurrence, and

R is H, $C_1$—$C_4$ alkyl or a pharmaceutically acceptable table metal or ammonium cation.

Those of the preceding compounds in which X is H are preferred; particularly preferred among the latter compounds is the one in which R is $Na^+$, in both occurrences.

The subject compounds may be more formally named as N,N'-(2,6-pyrazinediyl)bis(2-oxoglycines), as such, as their salts or as their dialkyl esters.

As employed herein, the term "pharmaceutically acceptable metal or ammonium cation" is intended to include non-toxic metal cationic salts such as the alkali metal salts, e.g., sodium and potassium, alkaline earth metal salts such as calcium, magnesium or barium; and salts with ammonia and amines, e.g., amines such as triethylamine, n-propylamine, tri-n-butylamine, tris(hydroxymethyl)aminomethane (THAM), N,N-bis(hydroxyethyl)piperazine, 2-amino-2-methyl-1-propanol, 2-amino-2-methyl-1,3-propanediol, and triethanolamine. The alkali metal salts and, particularly the sodium salt, are preferred.

DETAILED DESCRIPTION

The compounds of the present invention possess antiallergic activity. Thus, they are useful in the treatment of conditions in which antigen-antibody reactions are responsible for disease and particularly in the treatment of allergic diseases such as (but not limited to) asthma, hay fever, urticaria, eczema or atopic dermatitis and upper respiratory conditions such as allergic rhinitis.

The compounds of the present invention may be administered either as individual therapeutic agents or as mixtures with other therapeutic agents. They may be administered alone but are generally administered in the form of pharmaceutical compositions, i.e., mixtures of active agents with suitable pharmaceutical carriers or diluents. Examples of such compositions include tablets, lozenges, capsules, suppositories, powders, aerosol sprays, aqueous or oily suspensions, syrups, elixirs and aqueous solutions for injection. The compounds are most preferably administered in oral dosage forms.

The nature of the pharmaceutical composition and the pharmaceutical carrier or diluent will, of course, depend on the desired route of administration, i.e., orally, parenterally or by inhalation. Oral compositions may be in the form of tablets or capsules and may contain conventional excipients such as binding agents (e.g., syrup, acacia, gelatin, sorbitol, tragacanth or polyvinylpyrrolidone), fillers (e.g., lactose, sugar, maize-starch, calcium phosphate, sorbitol or glycine), lubricants (e.g., magnesium stearate, talc, polyethylene glycol, or silica), disintegrants (e.g., starch) or wetting agents (e.g., sodium lauryl sulfate). Oral liquid

preparations may be in the form of aqueous or oily suspensions, solutions, emulsions, syrups, elixirs, etc., or may be presented as a dry product for reconstitution with water or other suitable vehicle before use. Such liquid preparations may contain conventional additives such as suspending agents, flavoring agents, diluents or emulsifying agents. For parenteral administration or inhalation, solutions or suspensions of a compound of formula 1 with conventional pharmaceutical vehicles may be employed, e.g., as an aerosol spray for inhalation, as an aqueous solution for intravenous injection or as an oily suspension for intramuscular injection. The compounds may also be administered by means of inhalers or other devices which permit the active compounds in the form of dry powders to come into direct contact with the lungs. Standard procedures are used for the preparation of these compositions as described in U.S. 3,963,660.

The compounds of the present invention or pharmaceutical compositions may be administered to human asthmatic patients in single oral doses of approximately 1—1000 mg of active ingredient and multiple oral doses totalling up to about 4000 mg/day of active ingredient. When administered by inhalation, lower doses are generally given, i.e., on the order of about 0.1 of the normal dosage for the particular compounds in question. These values are illustrative only, however, and the physician of course will ultimately determine the dosage most suitable for a particular patient on the basis of factors such as age, weight, diagnosis, severity of the symptoms and the particular agent to be administered.

METHODS OF PREPARATION

To prepare the compounds of the present invention, a diaminopyrazine of the formula:

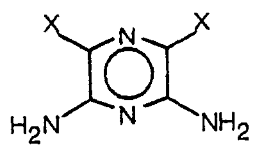

wherein X is H, Br or Cl, is used as the starting material. [Where the starting material is a 3,5-dihalo-2,6-diaminopyrazine, it can be obtained by the method disclosed in U.S.P. 3,987,044 — reaction of a tetrahalopyrazine with $NH_4OH$ under pressure and separation of the 2,3-diamino isomer from the resulting isomer mixture as a 1:1 association product with a ketone.] The indicated diamino starting material is reacted with a [$C_1$—$C_4$ alkyl] oxalyl halide of the formula

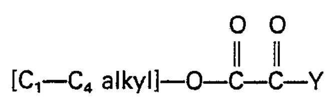

wherein Y is chlorine or bromine to give the esters of the present invention. When X is H, this can be followed by appropriate halogenation to give the esters wherein X is Br or Cl. Treatment of any of the resulting esters with an appropriate metal hydroxide gives the corresponding metal salt. Alkali metal hydroxides and, particularly, sodium hydroxide, are preferred for this hydrolysis. Further optional treatment of the metal salt with a strong inorganic acid gives the corresponding free carboxylic acid. Acids such as hydrochloric acid are preferred for this purpose. Reaction of the free carboxylic acid with ammonia or a pharmaceutically acceptable amine then gives the corresponding ammonium salt.

Although two different methods for the preparation of the halogen-substituted compounds of the present invention are actually included in the procedure described above, the preferred method is to convert the 3,5-dihalo-2,6-diamino intermediate to 2,6-diaminopyrazine before reacting the amino groups with an alkyl oxalyl halide, preferably the chloride. Thus, an alkyl oxalyl halide reacts readily with the first amino group in halogen-substituted diaminopyrazines but reaction with the second amino group occurs with difficulty. The process gives a mixture of the dioxamate with a relatively high proportion of the monooxamate and it is necessary to separate the mixture. However, 2,6-diaminopyrazine is readily converted to the dioxamate, which can be halogenated cleanly to give the 3,5-dihalo compound.

The sequences of reactions indicated above — in which the greater reactivity of the 2- and 6-amino groups in an otherwise unsubstituted pyrazine can be utilized in the preparation of a final product having halogens in the 3 and 5 positions — is an additional process aspect of the invention. This method of obtaining the 3,5-dihalo-2,6-dioxamic acid derivatives can be summarized by the following three steps: (1) hydrogenolysis of a 2,6-diamino-3,5-dihalopyrazine to replace the 3,5-halogens with hydrogen; (2) reacting the resulting 2,6-diaminopyrazine with an alkyl oxalyl halide (ethyl oxalyl chloride, preferably) to give the dioxamate; and, (3) halogenating the resulting 2,6-dioxamate to give the corresponding 3,5-dihalo compound. These steps constitute a novel sequence of reactions, said sequence being a process aspect of the present invention. (Although other methods for making various 2,6-dihalopyrazines are known, the preparation of the particular oxamide-substituted pyrazines of the present invention by the indicated process is not known, to the best of the present Applicant's knowledge). Thus, the present process provides a convenient synthesis of the indicated compounds when the tetrahalopyrazine is readily available as the starting material and it could also be used to obtain the dioxamates without the halogens.

METHOD OF ASSAY FOR ACTIVITY

The antiallergic activity of the present compounds is demonstrated by the IgE-mediated rat Passive Cutaneous Anaphylaxis (PCA) test. This test is generally accepted as one of the best animal models for the qualitative determination of antiallergic activity. Disodium cromoglycate is active in this test when administered i.p. but not orally. The method can be described briefly as follows:

*PCA Test Method*

1. Antisera — Various standard methods described in the literature were used for the preparation of reaginic antisera to ovalbumin in either Hooded Lister or Brown Norway adult rats.

2. Animals — Adult male Sprague-Dawley or female Wistar Kyoto rats were used as antisera recipients in the test. The animals were allowed to acclimate for 5—14 days with food and water *ad lib.*

3. Sensitization — Recipient rats were passively sensitized by the intradermal injection of 100 microliters of two dilutions of antiserum (one injection on each side of the back). Sensitization occurred 48—72 hours prior to antigen challenge.

4. Administration of Test Compound — Four to six animals were used for each test compound/dilution. Compounds were homogenized in an appropriate carrier solution, and administered i.p. 5 minutes prior to challenge or p.o. 5 to 240 minutes prior to challenge. In both cases, an appropriate range of doses was used.

5. Antigen Challenge and Reaction Evaluation — Ovalbumin (0.1—1.0 mg in a 0.5% solution of Evan's Blue dye) in saline was given to each rat by i.v. administration. Thirty (30) minutes later, the resultant PCA reactions were measured for average diameter and color intensity from the reflected surface of the skin. Test compound activity is expressed as percent inhibition based on control reactions.

When tested by the above procedure, the compounds of the present invention were active both i.p. and orally.

In addition to activity in the PCA test as described above, the compounds also inhibit the release of histamine in the rat Passive Peritoneal Anaphylaxis (PPA) test. This method can be described briefly as follows:

*PPA Test Method*

1. Antisera — Reaginic antibody to ovalbumin for this test was prepared in adult male $B_6D_2F_1$ mice.

2. Animals — Adult male Sprague Dawley or female Wistar Kyoto rats were used as antibody recipients. The animals were allowed to acclimate for 5—14 days with food and water *ad lib.*

3. Sensitization — Recipient rats were sensitized i.p. with 2 ml of an appropriate saline dilution of the mouse anti-ovalbumin antiserum determined from prior experiments. Sensitization took place 2 hours prior to antigen challenge.

4. Administration of Test Compound — Five to ten animals were used for each test compound/dilution. Compounds were homogenized in saline with an equivalent of base to effect solubilization, if appropriate, and administered i.p. 30 seconds prior to antigen challenge or p.o. 5 to 60 minutes prior to antigen challenge, using an appropriate range of doses.

5. Antigen Challenge and Assay Evaluation — Two (2) mg of ovalbumin in 5 ml of modified Tyrode's Solution was administered by i.p. injection and the animals were sacrificed 5 minutes later. Peritoneal shock fluids were collected and clarified by centrifugation. Protein was removed from the samples by perchloric acid precipitation and subsequent centrifugation. The samples were then analyzed for histamine content by an automated fluorometric assay. Histamine levels of peritoneal shock fluids from treatment animals were then compared to those of shock fluids from control animals. Drug effect was expressed as percent inhibition of histamine release.

Examples

The following examples are for purposes of illustration and are not to be construed as limiting the present invention in a manner inconsistent with the claims appended to these specifications.

*Preparations of Compounds*

Example 1

N,N'-(2,6-Pyrazinediyl)bis(2-oxoglycine), Diethyl Ester

2,6-Diaminopyrazine, 4.25 g (0.04 mole) was dissolved in 75 ml of THF and 8.7 ml (0.08 mole) of ethyl oxalyl chloride was added to the stirred mixture. When the reaction had subsided, 11 ml (0.08 mole) of triethylamine was slowly added to the reaction and the temperature was raised to reflux for two hours. At the end of this time, the THF was removed on a rotary evaporator and the residue was washed well with water followed by saturated $NaHCO_3$ solution. The resulting material was filtered and dried on a porous plate. The crude material was placed in a Soxhlet extraction apparatus and extracted with THF. The THF was removed on a rotary evaporator to give 7.1 g (60% of theory) of N,N'-(2,6-pyrazinediyl)bis(2-oxoglycine), diethyl ester as a white solid, m.p. 187—189°C.

| | | | |
|---|---|---|---|
| Calcd. for $C_{12}H_{14}N_4O_6$: | C, 46.45; | H, 4.55; | N, 18.06 |
| Found: | C, 46.47; | H, 4.55; | N, 17.92 |

The above compound has the following structural formula:

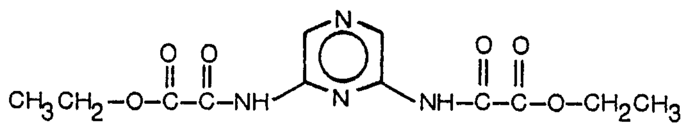

Example 2

N,N'-(3,5-Dichloro-2,6-pyrazinediyl)bis(2-oxoglycine), Diethyl Ester

2,6-Diamino-3,5-dichloropyrazine, 15 g (0.08 mole) was placed in 75 ml of THF and 25 ml (0.17 mole) of triethylamine was added to the stirred solution. Ethyl oxalyl chloride, 25 g (0.17 mole) was then added to the solution portion-wise. The reaction was allowed to reflux for two hours, cooled and the solvent was removed on a rotary avaporator. The residue was washed with water and taken up in dichloromethane. The solvent was dried, filtered and removed. The resulting oil slowly partially solidified. The solid was removed by filtration. Treatment of the filtrate with isopropyl alcohol/hexane resulted in more of originally-obtained solid. Treatment of the second filtrate with ether resulted in a second solid. The first solid was recrystallized from ethanol to yield white needles, 11.8 g, m.p. 156—157°C. It was found to be N-(6-amino-3,5-dichloropyrazinyl)-2-oxoglycine, ethyl ester.

| | | | |
|---|---|---|---|
| Calcd. for $C_8H_8Cl_2N_4O_3$ | C, 34.43; | H, 2.88; | N, 20.08 |
| Found: | C, 34.62; | H, 3.00; | N, 19.89 |

The second solid was determined to be N,N'-(3,5-dichloro-2,6-pyrazinediyl)bis(2-oxoglycine), diethyl ester. It was recrystallized from 1,1,1-trichloroethane and hexane. White crystals, 4.9 g, m.p. 115—117°C.

| | | | |
|---|---|---|---|
| Calcd. for $C_{12}H_{12}Cl_2N_4O_6$: | C, 38.01; | H, 3.19; | N, 14.78 |
| Found: | C, 37.99; | H, 3.22; | N, 14.76 |

Example 3

N,N'-(2,6-Pyrazinediyl)bis(2-oxoglycine), MonoSodium Salt

The diester (Example 1), 3.1 grams (10 mmole) was stirred in 40 ml of 1N NaOH (40 mequiv.) and chilled in ice water. The basic solution was then neutralized with 10 ml of 3N HCl (30 mequiv.). The resulting solid precipitate was collected by filtration, washed with dilute HCl, then with water, and dried under reduced pressure. The product [N,N'-(2,6-pyrazinediyl)bis(2-oxoglycine), monosodium salt], 2.5 grams, analyzed as follows.

| | | | |
|---|---|---|---|
| Calcd. for $C_8H_5N_4NaO_6.H_2O$: | C, 32.66; | H, 2.40; | N, 19.05 |
| Found: | C, 32.94; | H, 2.52; | N, 20.13 |

Example 4

N,N'-(2,6-Pyrazinediyl)bis(2-oxoglycine), Disodium Salt

The diester, 9.3 grams (30 mmole) was mixed with 40 ml of 1N NaOH (40 mmole) and 3.18 grams (30 mmole) of $Na_2CO_3$ in 30 ml of water and stirred at room temperature. The resulting pasty precipitate was filtered out, washed with water, alcohol and acetone and dried in vacuo. The product [N,N'-(2,6-pyrazinediyl)bis(2-oxoglycine), disodium salt], 9.2 grams, analyzed as follows.

| | | | |
|---|---|---|---|
| Calcd. for $C_8H_4N_4Na_2O_6.2H_2O$: | C, 28.75; | H, 2.41; | N, 16.77 |
| Found: | C, 28.14; | H, 2.63; | N, 16.94 |

Example 5

N,N'-(2,6-Pyrazinediyl)bis(2-oxoglycine) (The Free Bis-Carboxylic Acid)

4.65 Grams (15 mmole) of the diester was stirred with 60 ml of 1N NaOH (60 mequiv.) and chilled in ice water. The basic solution was neutralized with 20 ml of 3N HCl (60 mequiv.). The resulting solid precipitate was filtered out and dried in vacuo. The (hygroscopic) product [N,N'-(2,6-pyrazinediyl)bis(2-oxoglycine) (the free bis-carboxylic acid)], 3.6 grams, analyzed as follows.

| | | | |
|---|---|---|---|
| Calcd. for $C_8H_6N_4O_6$: | C, 37.80; | H, 2.38; | N, 22.05 |
| Found: | C, 36.37; | H, 2.87; | N, 22.57 |

Example 6

N,N'-(2,6-Pyrazinediyl)bis(2-oxoglycine), Salt With Two Molecular Proportions of Tris(hydroxymethyl)aminomethane (THAM)

The diester, 4.65 grams (15 mmole), was converted to the diacid as per Example 5. It was then suspended in water and mixed well with 3.37 grams (28 mmole) of THAM. The solution was freeze-dried and the resulting residue stirred in absolute alcohol; the mixture was diluted with anhydrous diethyl ether and filtered. The solid obtained was dried in vacuo overnight. The (hygroscopic) product [N,N'-(2,6-pyrazinediyl)bis(2-oxoglycine), salt with two molecular proportions of tris(hydroxymethyl)aminomethane (THAM)], 6.44 grams, analyzed as follows.

| | | | |
|---|---|---|---|
| Calcd. for $C_{16}H_{28}N_6O_{12}.2H_2O$: | C, 36.09; | H, 6.06; | N, 15.78 |
| Found: | C, 36.05; | H, 5.81; | N, 15.72 |

Example 7

Alternative Preparation of N,N'-(3,5-Dichloro-2,6-pyrazinediyl)bis(2-oxoglycine), Diethyl Ester

*Step 1*

A solution of 26.8 grams (0.15 mole) of 2,6-diamino-3,5-dichloropyrazine and 19.8 grams of 85% KOH (0.30 mole) in 200 ml of ethanol was placed in a Parr hydrogenation bottle. Three grams of 5% palladium on charcoal catalyst was added and the bottle pressurized with hydrogen gas and shaken at room temperature until hydrogen consumption stopped. The catalyst and salt solids were filtered out and the filtrate evaporated to drynes. The residue was a crystalline solid, 16.1 grams of 2,6-diaminopyrazine.

*Step 2*

The product of Step 1 was reacted with ethyloxalyl chloride in the manner of Example 1.

*Step 3*

The product of Step 2 [(N,N'-(2,6-pyrazinediyl)-bis(2-oxoglycine)], in the amount of 10 grams, was mixed with 150 ml of acetonitrile and warmed to 60°C. Chlorine gas was bubbled into the mixture, with stirring. A mildly exothermic reaction ensued and the starting material dissolved. After chlorination had continued beyond this point for 40 minutes, chlorine introduction was stopped. Analysis of a sample of the reaction mixture by TLC (thin layer chromatography) showed the reaction was complete. The solid obtained upon acetonitrile removal (in a rotary evaporator) was recrystallized to yield about 7 grams (only one crop taken) of product [N,N'-(3,5-dichloro-2,6-pyrazinediyl)bis(2-oxo-glycine), diethyl ester by nuclear magnetic resonance and infrared analyses)]. Elemental analysis gave the following results.

| | | | |
|---|---|---|---|
| Calcd. for $C_{12}H_{12}Cl_2N_4O_6$: | C, 38.01; | H, 3.19; | N, 14.78 |
| Found: | C, 38.05; | H, 3.05; | N, 14.97 |

Example 8

Following the procedures as described in Examples 1—7 and using the appropriate reactants, the following additional compounds can be obtained:

N,N'-(2,6-Pyrazinediyl)bis(2-oxoglycine), dimethyl ester.

N,N'-(2,6-Pyrazinediyl)bis(2-oxoglycine), dibutyl ester.

N,N'-(3,5-Dibromo-2,6-pyrazinediyl)bis(2-oxoglycine), diethyl ester.

N,N'-(2,6-Pyrazinediyl)bis(2-oxoglycine), di-potassium salt.

N,N'-(2,6-Pyrazinediyl)bis(2-oxoglycine), di-ammonium salt.

**Claims for the Contracting States: BE CH DE FR GB IT LI LU NL SE**

1. A compound of the formula:

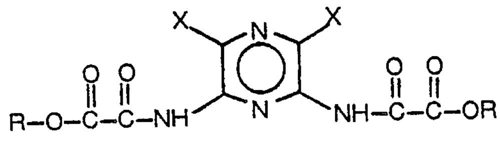

wherein X is H, Br or Cl, and R is H, $C_1$—$C_4$ alkyl or a pharmaceutically acceptable metal or ammonium cation.

2. A compound according to Claim 1 which has the formula:

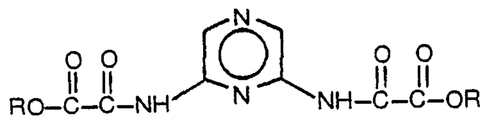

wherein R is H, $C_1$—$C_4$ alkyl or a pharmaceutically acceptable metal or ammonium cation.

3. A compound according to Claim 1 which has the formula:

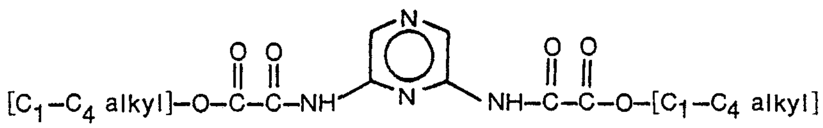

4. A compound according to Claim 1 which is N,N'-(2,6-pyrazinediyl)bis(2-oxoglycine), diethyl ester.

5. A compound according to Claim 1 which has the formula:

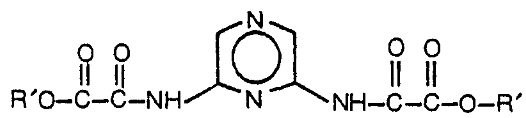

wherein R' is H or a pharmaceutically acceptable metal or ammonium cation.

6. A compound according to Claim 1 which is N,N'-(2,6-pyrazinediyl)bis(2-oxoglycine), disodium salt.

7. A process for preparing a compound of the formula:

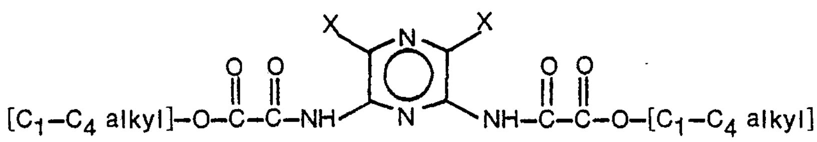

wherein X is Br or Cl, which comprises

a) Catalytic hydrogenation of a 2,6-diamino-3,5-di(Cl or Br)-pyrazine to give 2,6-diaminopyrazine;

b) Reaction of the 2,6-diaminopyrazine with a $C_1$—$C_4$ alkyl oxalyl chloride to give the corresponding 2,6-bis-(2-oxoglycine) ester;

c) Halogenation of the resulting ester with chlorine or bromine to give the desired product.

8. A process according to Claim 7 wherein X is Cl and the halogenating agent is chlorine.

9. A process according to Claim 7 wherein the product obtained is further reacted with an alkali metal base to give the corresponding bis salt.

10. A process according to Claim 9 wherein the base used is sodium hydroxide and the disodium salt is obtained as the product.

**Claims for the Contracting State: AT**

1. A process for preparing a compound of the formula:

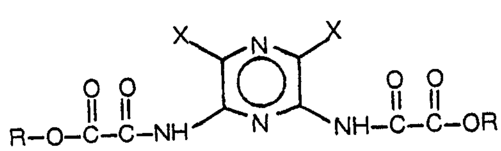

wherein X is H, Br or Cl, and R is H, $C_1$—$C_4$ alkyl or a pharmaceutically acceptable metal or ammonium cation which comprises:

(a) reacting a diaminopyrazine of the formula:

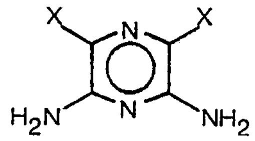

wherein X is defined as above, with a [$C_1$—$C_4$ alkyl] oxalyl halide of the formula:

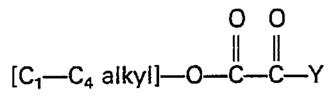

wherein Y is chlorine or bromine to give the esters of the formula:

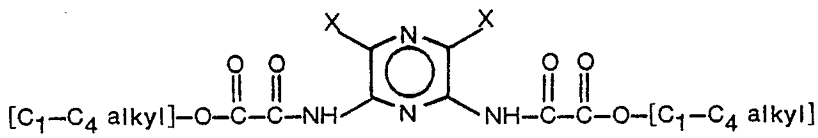

(b) optionally followed, when X is H, by appropriate halogenation to give the esters wherein X is Br or Cl;

(c) optionally followed by treatment of the ester with an appropriate metal hydroxide to give the corresponding metal salt;

(c) further optionally followed by treatment of the metal salt with a strong inorganic acid to give the corresponding free carboxylic acid;

(e) further optionally followed by reaction of the free carboxylic acid with ammonia or a pharmaceutically acceptable amine to give the corresponding ammonium salt.

2. A process according to Claim 1 for preparing a compound of the formula:

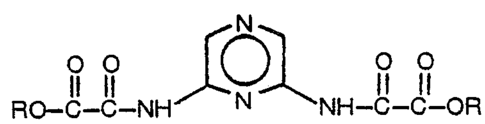

wherein R is H, $C_1$—$C_4$ alkyl or a pharmaceutically acceptable metal or ammonium cation which comprises:

(a) reacting 2,6-diaminopyrazine with a [$C_1$—$C_4$ alkyl] oxalyl halide of the formula:

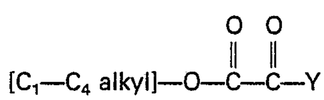

wherein Y is chlorine or bromine to give the esters of the formula:

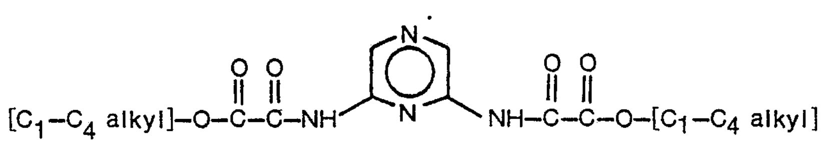

(b) optionally followed by treatment of the ester with an appropriate metal hydroxide to give the corresponding metal salt;

(c) further optionally followed by treatment of the metal salt with a strong inorganic acid to give the corresponding free carboxylic acid;

(d) further optionally followed by reaction of the free carboxylic acid with ammonia or a pharmaceutically acceptable amine to give the corresponding ammonium salt.

3. A process according to Claim 1 for preparing a compound of the formula

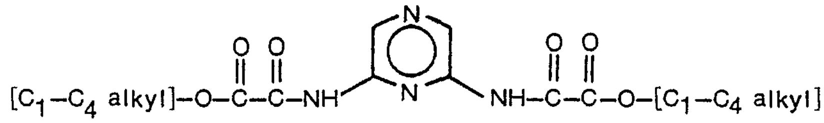

which comprises reacting 2,6-diaminopyrazine with a [$C_1$—$C_4$ alkyl] oxalyl halide of the formula:

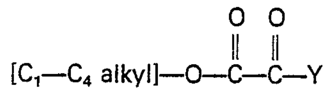

wherein Y is chlorine or bromine.

4. A process according to Claim 1 for preparing N,N'-(2,6-pyrazinediyl)bis(2-oxoglycine), diethyl ester which comprises reacting 2,6-diaminopyrazine with ethyl oxalyl chloride.

5. A process according to Claim 1 for preparing a compound of the formula:

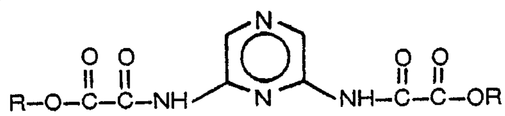

wherein R is a pharmaceutically acceptable metal or ammonium cation which comprises:

(a) reacting 2,6-diaminopyrazine with a [$C_1$—$C_4$ alkyl] oxalyl halide of the formula:

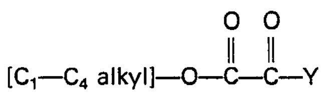

wherein Y is chlorine or bromine to give the corresponding esters, followed by treatment of the ester with an appropriate metal hydroxide to give the corresonding metal salt,

(b) optionally followed by treatment of the metal salt with a strong inorganic acid to give the corresponding free carboxylic acid, and further followed by reaction of the free carboxylic acid with ammonia or a pharmaceutically acceptable amine to give the corresponding ammonium salt.

6. A process according to Claim 1 for preparing N,N'-(2,6-pyrazinediyl)bis(2-oxoglycine), disodium salt which comprises reacting 2,6-diaminopyrazine with ethyl oxalyl chloride to give N,N'-(2,6-pyrazinediyl)bis(2-oxoglycine), diethyl ester followed by reaction with aqueous sodium hydroxide.

**Patentansprüche für die Vertragsstaaten: BE CH DE FR GB IT LI LU NL SE**

1. Verbindung der allgemeinen Formel

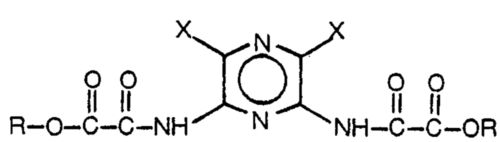

in der X ein Wasserstoff-, Brom- oder Chloratom bedeutet, und R ein Wasserstoffatom, einen $C_1$—$C_4$-Alkylrest oder ein pharmakologisch verträgliches Metall- oder Ammoniumkation bedeutet.

2. Verbindung nach Anspruch 1, mit der allgemeinen Formel

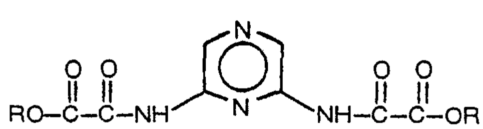

in der R ein Wasserstoffatom, einen $C_1$—$C_4$-Alkylrest oder ein pharmakologisch verträgliches Metall- oder Ammoniumkation bedeutet.

3. Verbindung nach Anspruch 1, mit der folgenden allgemeinen Formel

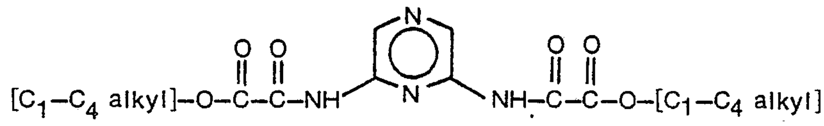

4. Verbindung nach Anspruch 1, nämlich N,N'-(2,6-Pyrazindiyl)-bis-(2-oxoglycin)-diäthylester.

5. Verbindung nach Anspruch 1, mit der allgemeinen Formel

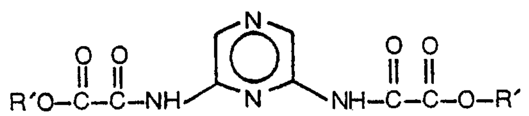

in der R' ein Wasserstoffatom oder ein pharmakologisch verträgliches Metall- oder Ammoniumkation bedeutet.

6. Verbindung nach Anspruch 1, nämlich N,N'-(2,6-Pyrazindiyl)-bis-(2-oxoglycin)-dinatriumsalz.

7. Verfahren zur Herstellung einer Verbindung der allgemeinen Formel:

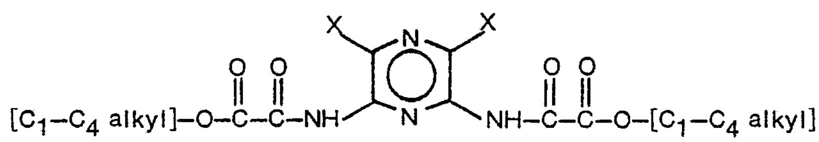

in der X ein Chlor- oder Bromatom bedeutet, kennzeichnet durch folgende Schritte:

a) katalytische Hydrierung von 2,6-Diamino-3,5-di(Cl oder Br)-pyrazin zu 2,6-Diaminopyrazin,

b) Umsetzung des 2,6-Diaminopyrazins mit einem $C_1$—$C_4$-Alkyloxalylchlorid zu dem entsprechenden 2,6-Bis-(2-oxoglycin)-ester,

c) Halogenierung des erhaltenen Esters mit Chlor oder Brom zum gewünschten Produkt.

8. Verfahren nach Anspruch 7, in dem X ein Chloratom bedeutet und das Halogenierungsmittel Chlor ist.

9. Verfahren nach Anspruch 7, in dem das erhaltene Produkt mit einer Alkalimetallbase zu dem entsprechenden Bis-Salz weiter umgesetzt wird.

10. Verfahren nach Anspruch 9, in dem die verwendete Base Natriumhydroxid ist und das Dinatriumsalz als Produkt erhalten wird.

**Patentansprüche für den Vertragsstaat: AT**

1. Verfahren zur Herstellung einer Verbindung der allgemeinen Formel:

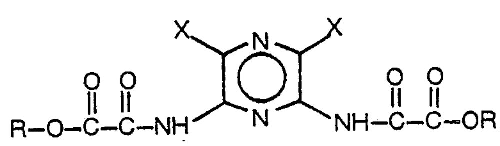

in der X ein Wasserstoff-, Brom- oder Chloratom bedeutet, und R ein Wasserstoffatom, einen $C_1$—$C_4$-Alkylrest oder ein pharmakologisch verträgliches Metall- oder Ammoniumkation darstellt, gekennzeichnet durch folgende Schritte:

(a) Umsetzung eines Diaminopyrazins der allgemeinen Formel:

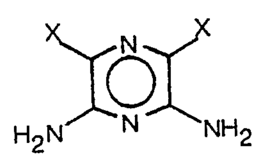

in der X die vorstehend angegebene Bedeutung hat, mit einem ($C_1$—$C_4$-Alkyl)-oxalylhalogenid der allgemeinen Formel

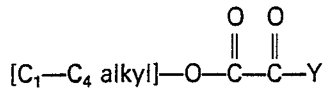

in der Y ein Chlor- oder Bromatom bedeutet, zu den Estern der allgemeinen Formel:

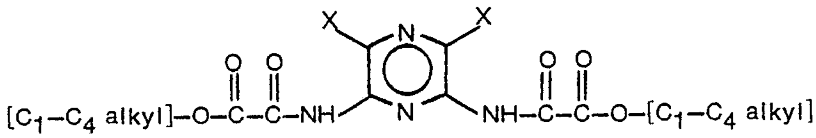

(b) wenn X ein Wasserstoffatom bedeutet, gegebenenfalls gefolgt von einer geeigneten Halogenierung zu den Estern, in denen X ein Bromatom oder Chloratom darstellt,

(c) gegebenenfalls gefolgt von einer Behandlung des Esters mit einem geeigneten Metallhydroxid zu dem entsprechenden Metallsalz,

(d) gegebenenfalls gefolgt von einer Behandlung des Metallsalzes mit einer starken anorganischen Säure zu der entsprechenden freien Carbonsäure,

(e) gegebenenfalls gefolgt von einer Umsetzung der freien Carbonsäure mit Ammoniak oder einem pharmakologisch verträglichen Amin zu dem entsprechenden Ammoniumsalz.

2. Verfahren nach Anspruch 1 zur Herstellung einer Verbindung der allgemeinen Formel:

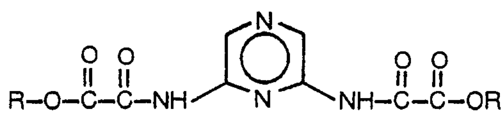

in der R ein Wasserstoffatom, einen $C_1$—$C_4$-Alkylrest oder ein pharmakologisch verträgliches Metall- oder Ammoniumkation bedeutet, gekennzeichnet durch folgende Schritte:

(a) Umsetzung von 2,6-Diaminopyrazin mit einem ($C_1$—$C_4$-Alkyl)-oxalylhalogenid der allgemeinen Formel:

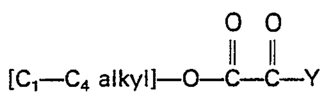

in der Y ein Chlor- oder Bromatom bedeutet, zu den Estern der allgemeinen Formel:

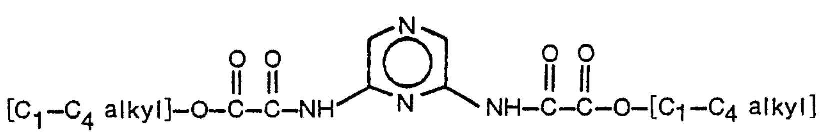

(b) gegebenenfalls gefolgt von einer Behandlung des Esters mit einem geeigneten Metallhydroxid zu dem entsprechenden Metallsalz.

(c) gegebenenfalls gefolgt von einer Behandlung des Metallsalzes mit einer starken anorganischen Säure zu der entsprechenden freien Carbonsäure,

(d) gegebenenfalls gefolgt von einer Umsetzung der freien Carbonsäure mit Ammoniak oder einem pharmakologisch verträglichen Amin zu dem entsprechenden Ammoniumsalz.

3. Verfahren nach Anspruch 1 zur Herstellung einer Verbindung der allgemeinen Formel:

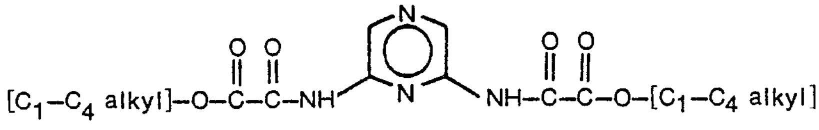

gekennzeichnet durch die Umsetzung von 2,6-Diaminopyrazin mit einem ($C_1$—$C_4$-Alkyl)-oxalylhalogenid der allgemeinen Formel

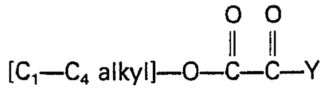

in der Y ein Chlor- oder Bromatom bedeutet.

4. Verfahren nach Anspruch 1 zur Herstellung von N,N'-(2,6-Pyrazindiyl)-bis-(2-oxoglycin)-diäthylester,

gekennzeichnet durch die Umsetzung von 2,6-Diaminopyrazin mit Äthyloxalylchlorid.

5. Verfahren nach Anspruch 1 zur Herstellung einer Verbindung der allgemeinen Formel:

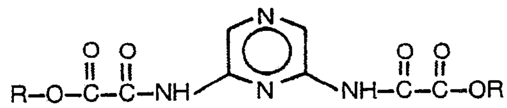

in der R ein pharmakologisch verträgliches Metall- oder Ammoniumkation bedeutet, gekennzeichnet durch folgende Schritte:

(a) Umsetzung von 2,6-Diaminopyrazin mit einem ($C_1$—$C_4$-Alkyl)-oxalylhalogenid der allgemeinen Formel:

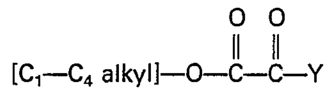

in der Y ein Chlor- oder Bromatom bedeutet zu den entsprechenden Estern, gefolgt von einer Behandlung des Esters mit einem geeigneten Metallhydroxid zu dem entsprechenden Metallsalz.

(b) gegebenenfalls gefolgt von einer Behandlung des Metallsalzes mit einer starken anorganischen Säure zu der entsprechenden freien Carbonsäure, und weiterhin gefolgt von der Umsetzung der freien Carbonsäure mit Ammoniak oder einem pharmakologisch verträglichen Amin zu dem entsprechenden Ammoniumsalz.

6. Verfahren nach Anspruch 1 zur Herstellung von N,N'-(2,6-Pyrazindiyl)-bis-(2-oxoglycin)-dinatriumsalz, gekennzeichnet durch die Umsetzung von 2,6-Diaminopyrazin mit Äthyloxalylchlorid zu N,N'-(2,6-Pyrazindiyl)-2-bis-(2-oxoglycin)-diäthylester und anschließende Umsetzung mit wäßrigem Natriumhydroxid.

**Revendication pour les Etats contractants: BE CH DE FR GB IT LI LU NL SE**

1. Composé de formule:

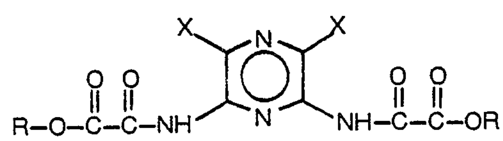

dans laquelle X est H, Br ou Cl; et R est H, un groupe alkyle en $C_1$—$C_4$, ou un métal pharmaceutiquement acceptable ou un cation ammonium.

2. Composé selon la revendication 1, lequel possède la formule:

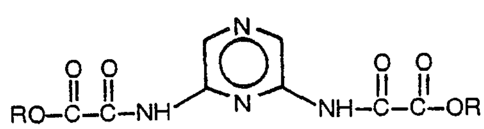

dans laquelle R est H, un groupe alkyle en $C_1$—$C_4$ ou un métal pharmaceutiquement acceptable ou un cation ammonium.

3. Composé selon la revendication 1, lequel possède la formule:

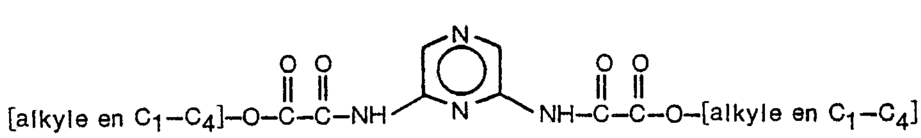

4. Composé selon la revendication 1, lequel est l'ester diéthylique de la N,N'-(2,6-pyrazinediyl)bis(2-oxoglycine).

5. Composé selon la revendication 1, lequel possède la formule:

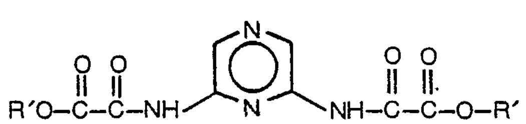

dans laquelle R' est H ou un métal pharmaceutiquement acceptable ou le cation ammonium.

6. Composé selon la revendication 1, lequel est le sel disodique de la N,N'-(2,6-pyrazinediyl)bis(2-oxoglycine).

7. Procédé pour la préparation d'un composé de formule:

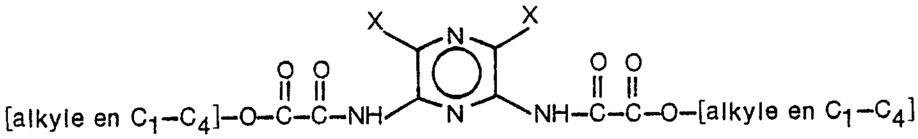

dans laquelle X est Br ou Cl, lequel comprend

a) l'hydrogénation catalytique d'une 2,6-diamino-3,5-di(Cl ou Br)-pyrazine, pour obtenir la 2,6-diaminopyrazine;

b) la réaction de la 2,6-diaminopyrazine avec un chlorure d'alkyl($C_1$—$C_4$)-oxalyle, pour obtenir le 2,6-bis[ester de 2-oxoglycine] correspondant;

c) l'halogénation de l'ester résultant avec le chlore ou le brome, pour obtenir le produit recherché.

8. Procédé selon la revendication 7, dans lequel X est Cl et l'agent d'halogénation est le chlore.

9. Procédé selon la revendication 7, dans lequel on fait réagir à nouveau le produit obtenu avec une base d'un métal alcalin, pour obtenir le bi-sel correspondant.

10. Procédé selon la revendication 9, dans lequel la base utilisée est l'hydroxyde de sodium et le sel disodique est obtenu en tant que produit.

**Revendications pour l'Etat contractant: AT**

1. Procédé pour la préparation d'un composé de formule:

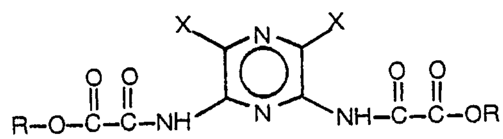

dans laquelle X est H, Br ou Cl; et R est H, un groupe alkyle en $C_1$—$C_4$, ou un métal pharmaceutiquement acceptable ou un cation ammonium, lequel comprend

(a) la réaction d'une diaminopyrazine de formule

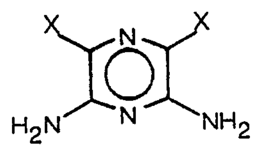

dans laquelle X est tel que défini plus haut, avec un halogénure d'alkyl($C_1$—$C_4$)-oxalyle de formule

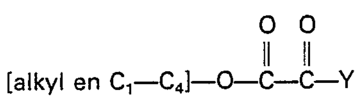

dans laquelle Y est le chlore ou le brome, pour obtenir les esters de formule

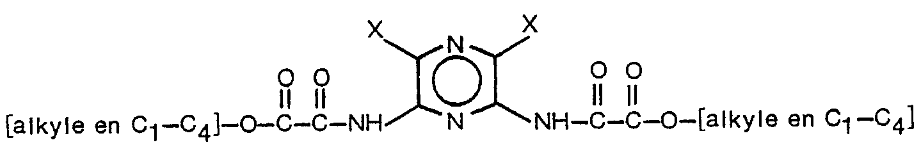

(b) éventuellement suivie, lorsque X est H, d'une halogénation appropriée, pour obtenir les esters dans lesquels X est Br ou Cl;

(c) éventuellement suivie, d'un triatement de l'ester avec un hydroxyde d'un métal approprié, pour obtenir le sel métallique correspondant;

(d) encore suivie éventuellement du traitement du sel métallique avec un acide minéral fort, pour obtenir l'acide carboxylique libre correspondant;

(e) encore suivie éventuellement d'une réaction de l'acide carboxylique libre avec l'ammoniac ou une amine pharmaceutiquement acceptable, pour obtenir le sel d'ammonium correspondant.

2. Procédé selon la revendication 1, pour la préparation d'un composé de formule:

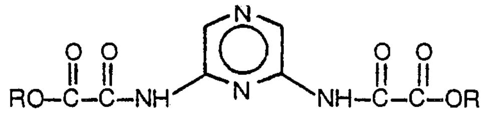

dans laquelle R est H, un groupe alkyle en $C_1$—$C_4$ ou un métal pharmaceutiquement acceptable ou un cation ammonium, lequel comprend

(a) la réaction de la 2,6-diaminopyrazine avec un halogénure d'alkyl($C_1$—$C_4$)-oxalyle de formule

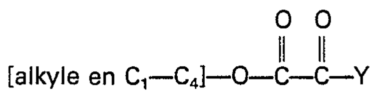

dans laquelle Y est le chlore ou le brome, pour obtenir les esters de formule

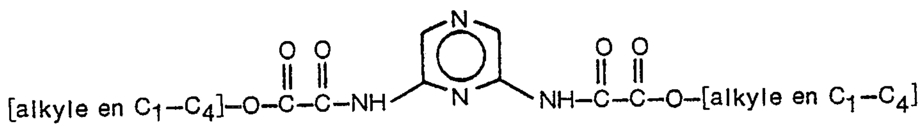

(b) éventuellement suivie du traitement de l'ester avec un hydroxyde d'un métal approprié, pour obtenir le sel métallique correspondant;

(c) encore suivie éventuellement d'un traitement du sel métallique avec un acide minéral fort, pour obtenir l'acide carboxylique libre correspondant;

(d) encore suivie éventuellement d'une réaction de l'acide carboxylique libre avec l'ammoniac ou une amine pharmaceutiquement acceptable, pour obtenir le sel d'ammonium correspondant.

3. Procédé selon la revendication 1, pour la préparation d'un composé de formule:

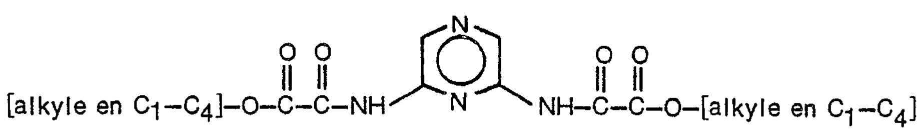

lequel comprend la réaction de la 2,6-diaminopyrazine avec un halogénure d'alkyl($C_1$—$C_4$)-oxalyle de formule

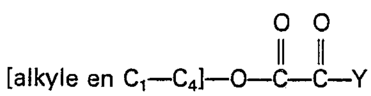

dans laquelle Y est le chlore ou le brome.

4. Procédé selon la revendication 1, pour la préparation de l'ester diéthylique de la N,N'-(2,6-pyrazinediyl)bis(2-oxoglycine), lequel comprend la réaction de la 2,6-diaminopyrazine avec le chlorure d'éthyloxalyle.

5. Procédé selon la revendication 1, pour la préparation d'un composé de formule

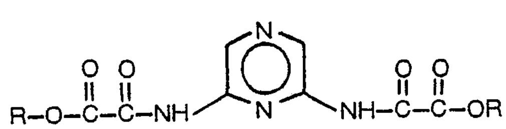

dans laquelle R est un métal pharmaceutiquement acceptable ou un cation ammonium, lequel comprend:

(a) la réaction de la 2,6-diaminopyrazine avec un halogénure d'alkyl($C_1$—$C_4$)-oxalyle de formule

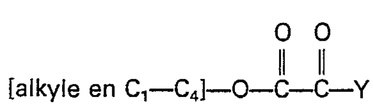

dans laquelle Y est le chlore ou le brome, pour obtenir les esters correspondants, suivie d'un traitement de l'ester avec un hydroxyde d'un métal approprié, pour obtenir le sel métallique correspondant;

(b) éventuellement suivie d'un traitement du sel métallique avec un acide minéral fort, pour obtenir

l'acide carboxylique libre correspondant, et encore suivie éventuellement d'une réaction de l'acide carboxylique libre avec l'ammoniac ou une amine pharmaceutiquement acceptable, pour obtenir le sel d'ammonium correspondant.

6. Procédé selon la revendication 1 pour la préparation du sel disodique de la N,N'-(2,6-pyrazinediyl)bis(2-oxoglycine), lequel comprend la réaction de la 2,6-diaminopyrazine avec le chlorure d'éthyloxalyle, pour obtenir l'ester diéthylique de la N,N'-(2,6-pyrazinediyl)bis(2-oxoglycine), suivie d'une réaction avec une solution aqueuse d'hydroxyde de sodium.